# EUROPEAN PATENT APPLICATION

(11) **EP 2 218 437 A1**
(43) Date of publication of application: **18.08.2010**
(21) Application number: 09153050.1
(22) Date of filing: 17.02.2009
(51) Int. Cl.: A61K 8/25, A61K 8/46, A61K 8/49, A61Q 9/02

(54) **Shaving compositions comprising dye-loaded particles**

(71) Applicant: The Gillette Company, Boston, Massachusetts 02199 (US)
(72) Inventor: Stephens, Alison Fiona, Maidenhead, Berkshire SL6 9LA (GB)
(74) Representative: Wilding, Richard Alan

(57) **Abstract**

According to the invention, a non-foaming shaving composition is provided comprising:
(i) Particles, each particle comprising at least one dye encapsulated by at least one siliceous encapsulating agent; and
(ii) A dermatologically acceptable carrier

## Description

### FIELD OF THE INVENTION

The present invention relates to coloured shaving formulations containing particles comprising encapsulated dyes.

### BACKGROUND TO THE INVENTION

A desirable aspect of a shaving composition is its ability to demonstrate to the user which regions of the skin have been shaven and which have not, a feature that shall be referred to herein as the ability to "track" where shaving has occurred and where not, or simply "tracking". Commercially available shaving compositions may be provided in the form of foam which facilitates tracking, but non-foaming compositions, such as shaving oils, are relatively difficult to see on the skin, making tracking difficult.

Tracking may be facilitated by colouring the shaving composition, coloured areas being indicative of where shaving has not yet occurred and non-coloured areas indicating where shaving is required. Metal oxide pigment particles may be incorporated within the shaving composition to achieve this However, pigments are only available in limited colours and do not provide good colour potency.

In order to overcome the limited colour palette available as metal oxide pigments, one might consider adding free organic dye - appropriate use of primary colours should enable the production of almost any other colour within the formulation, formulation considerations permitting. The use of organic dye may have disadvantages, however, not least of which is that dyes may stain the skin. This, in turn may give rise to safety concerns, especially if the composition is to be used around the eyes.

This problem is recognised in the art, where efforts are documented to immobilise the dyes within cosmetic formulations to prevent them from having adverse effects on skin. Reference can be made to the solid shaving compositions described in US 4,381,293, which teaches to mitigate skin staining by semi-permanently bonding the dyes to a polymer or using pigments as mentioned above. A disadvantage of the former approach is that the dyes must semi-permanently bind to the polymer molecules, limiting the range of available dyes.

An alternative approach to mitigate the problems associated with the use of free organic dye is to encapsulate them in other materials. WO2004/075679 describes a variety of compositions incorporating particles of dye encapsulated in an organic polymer. According to WO2004/075679, these particles may be used to provide a natural, textured tone effect to the skin. It is stated that they may also be used in shaving compositions, although the problem of tracking is not mentioned. Indeed, the purpose of achieving a natural, textured skin tone may be regarded as being contrary to the object of tracking as the former is a subtle effect not intended to substantially alter the colouration of the skin. Furthermore, the polymeric particles may demonstrate limited durability in the high shear conditions of shaving.

Accordingly, there is a need for shaving compositions which overcome the problems with prior art compositions.

### SUMMARY OF THE INVENTION

According to a first aspect of the invention, a non-foaming shaving composition is provided comprising:
(i) Particles, each particle comprising at least one dye encapsulated by at least one siliceous encapsulating agent; and
(ii) A dermatologically acceptable carrier

According to a second aspect of the invention, a shaving method is provided comprising the following steps:
(a) topically applying a non-foaming shaving composition according to the first aspect of the invention to an area of skin to be shaved;
(b) identifying areas of skin that have not been shaved by means of the colour bestowed by the particles;
(c) shaving identified areas of skin which have not been shaved.

According to a third aspect of the invention, a shaving device is provided, the shaving device comprising a reservoir containing the formulation according to the first aspect of the invention and means for dispensing the formulation onto the skin of a user.

### BRIEF DESCRIPTION OF THE DRAWINGS

Reference is made to the following drawings, which disclose aspects of a method for making particles which may be incorporated into the non-foaming shaving composition of the first aspect of the invention:
Figure 1 is a schematic representation of an apparatus suitable for the production of such particles.
Figure 2 is a schematic representation of the nozzle used in the apparatus shown in Fig. 1.
Figure 3 is the calibration for ion-exchanged Tartrazine in water at 423 nm.
Figure 4 shows the tartrazine leakage obtained in Example 4B.

### DETAILED DESCRIPTION OF THE INVENTION

The dimensions and values disclosed herein are not to be understood to be strictly limited to the exact numerical values recited. Instead, unless otherwise stated, each dimension is intended to mean both the recited value and a functionally equivalent range surrounding that value. For example, a dimension disclosed as "40nm" is intended to mean "about 40nm".

As used herein, the term "non-foaming" includes compositions comprising less than 2% of anionic surfactant, by weight of the composition, preferably less than 1%. More preferably, the term "non-foaming" refers to compositions comprising no anionic surfactant.

As used herein, the term "encapsulation" includes particles in which the dye is fully surrounded or encased by an encapsulating agent and, thus, held securely within the particle. Leakage of less than 5 weight %, preferably less than 2 weight %, more preferably less than 1 weight % of the total amount of dye incorporated into the particle is achieved, as determined by the methodology described herein in Example 4.

In order to improve particulate durability, it is possible to employ particles comprising a core of dye encapsulated by a shell (so-called "core-shell" particles) of a durable material, such as silica. WO 00/09652, for example, discloses dye encapsulated within a silica shell, made using a sol-gel process. The section from page 5, from immediately after "DETAILED DESCRIPTION OF THE INVENTION", to page 8, immediately above "EXAMPLES", which teaches how to make such particles, is incorporated herein by reference. This document does not disclose the use of such particles in a shaving context, nor does it refer to the problem of tracking, Furthermore, a disadvantage of the so-called "core-shell" materials disclosed in WO 00/09652, is that they offer limited colour efficiency on account of the dye being concentrated in the centre of the particles, so that parts of each particle do not contribute to colouration.

An alternative to "core-shell" type encapsulation may be provided by spray-dried particles produced according to the technique described in European patent applications 2007114266.1 and 2007114268.1 (both filed 13 August 2007), which technique produces particles encapsulating a homogenous distribution of dye throughout a siliceous encapsulating agent. These particles may provide improved colour efficiency versus "core-shell" type particles:

The amount of dye included in the particles can be varied in accordance with the desired applications or effects of the particles, and may also depend upon the type(s) of dye chosen to be included within the particles. Within the particles, the proportion of dye(s) is from 3% to 20%, by weight of the particle, Preferably, the proportion of dye is in the range from 5% to 15%, and more preferably from 8 to 12%, by weight of the particle.

The particles have a volume average particle size which renders them not discernible to the naked eye. Thus, such particles will typically have an average size of less than about 70 µm. For shaving applications, the average particle size is generally in the range of greater than 0 to 10 µm, preferably in the range of greater then 0 to 5 µm, more preferably from greater than 0 to less than 1 µm and even more preferably, is from 10nm to less than 1µm. The average particle size of the particles is measured using standard techniques of the art, such as light scattering *via* use of a Malvern Sizer 2000 apparatus or by scanning electron microscopy (SEM).

Particles comprising encapsulated dye may be present in the non-foaming shaving composition according to the invention in a concentration from 0.05% to 10%, by weight of the composition. Preferably, the particles are present in a concentration from 0.1% to 5%, more preferably 0.1% to 2% by weight of the non-foaming shaving composition.

Turning to the dyes encapsulated within the particles, a wide variety of dyes is suitable for this purpose. As used herein, the term "dye" includes any dye or colorant, which is desired to be introduced into a particle and indefinitely retained within that particle. Examples of dyes which may be comprises within the particles include, but are not limited to, dyes or colorants conventionally used in the end application(s) of choice. For example, the suitability of dyes for use in applications such as cosmetic, health, personal care and detergent compositions is governed by organizations such as the Food and Drug Administration (FDA) in the USA and equivalent bodies in other countries. Typically, suitable dyes may be cationic, anionic, neutral, amphoteric, zwitterionic or amphiphilic. The dyes are typically selected from conventionally-known dye types such as natural dyes, i.e. those derived from natural sources or synthetic equivalents thereof, azo dyes, indigoid dyes, triaryl-methane dyes, anthraquinone dyes, xanthine (xanthene) dyes, nitrosulphonate dyes, pyrene dyes, thiophene dyes, quinoline dyes and derivatives, lakes, composites or mixtures thereof, in particular those which have been approved for use by the FDA. Non-limiting examples of suitable dyes are provided in the following tablets (1 and 2).

| **Table 1: Colour Additives batch-certified by the FDA** | | |
|---|---|---|
| **Standard Name** | **Type** | **Colour Index Number (CI)** |
| FD&C OrangeNo.4 | monoazo | 15510 |
| FD&C Orange No. 5 | xanthene-based | 45370 |
| FD&C Orange No. 10 | xanthene-based | 45425 |
| FD&C Orange No. 11 (Sodium salt of Orange No. 10) | xanthene-based | 45425 |
| FD&C Blue No. 1 ("Erioglaucine") | triarylmethane | 42090 |
| FD&C Blue No. 4 | triarylmethane | 42090 |
| FD&C Brown No. 1 | diazo | 20170 |
| F&DC Violet No.2 | anthracene dione-based; i.e. anthraquinone-based | 60725 |
| Ext.D&CVioletNo.2 | anthracene-based | 60730 |
| FD&C Green No. 3 | triarylmethane | 42053 |
| FD&C Green No. 5 | anthracene-based | 61570 |
| FD&C Green No. 6 | anthracene-based | 61565 |
| FD&C Green No. 8 | pyrene-based | 59040 |
| FD&C Red No. 2 ("Amaranth") | monoazo | 16185 |
| FD&CRedNo.4 | monoazo | 14700 |
| FD&C Red No. 6 | monoazo | 15850 |
| FD&C Red No. 7 | monoazo | 15850 |
| FD&C Red No. 17 | diazo | 26100 |
| FD&C Red No. 21 | xanthene-based | 45380 |
| FD&C Red No. 22 | xanthene-based | 45380 |
| FD&C Red No. 27 | fluoran-based | 45410 |
| FD&C Red No. 28 | xanthene-based | 45410 |
| FD&C Red No. 30 | indigoid | 73360 |
| FD&C Red No. 31 | monoazo | 15800 |
| FD&C Red No. 33 | monoazo | 17200 |
| FD&C Red No. 34 | monoazo | 15880 |
| FD&C Red No. 40 | monoazo | 16035 |
| FD&C Yellow No. 5 ("Tartrazine") | monoazo | 19140 |
| FD&C Yellow No. 6 | monoazo | 15985 |
| FD&C Yellow No. 7 | xanthene-based | 45350 |
| Ext. D&C Yellow No.7 | dinitroarylsulphonate | 10316 |
| FD&C Yellow No. 8 | xanthene-based | 45350 |
| FD&C Yellow No. 10 | quinoline-based | 47005 |
| FD&C Yellow No. 11 | quinoline-based | 47000 |

| **Table 2: Natural Colour Additives Exempt from Batch Certification by the FDA** | |
|---|---|
| **Name** | **CI** |
| Caramel | n/a |
| Cochineal | 75470 |
| Beta carotene | 40800 or 75130 |
| Guanine | 75170 |
| Henna | n/a |

The following disclosure concerns particles made according to the methods disclosed in European patent applications 2007114266.1 and 2007114268.1. Both the particles and the encapsulating agent comprised within such particles are amorphous. As used herein, the term "amorphous" includes particles in which there is no long range crystallographic order in one, two or three dimensions at lengths from 0.1 - 50nm, as determined in the following way by a combination of powder x-ray diffraction (XRD) on bulk samples and transmission electron microscopy (TEM) of representative portions of the same bulk sample:
(a) The presence or a broad peak in the x-ray diffractogram centered between 2 theta angles corresponding to d-spacings of 0.37-0.42 nm, with full width half-maximum (FWHM) of between 5-10 degrees 2 theta;
(b) The lack of sharp powder x-ray diffraction peaks corresponding to spacings of crystallographic planes separated by 0,37-0.42nm;
(c) The lack of mesocrystalline order (where respective highest order Bragg peaks fall in the range 2-50nm - typical of ordered mesostructured materials), as determined by TEM imaging of samples prepared by microtoming;
(d) The lack of a multiplicity of sharp peaks in the range of two theta angles corresponding to d-spacings to 0.1-50nm.

This definition excludes ordered mesoporous materials with pore sizes from 2-50nm arranged with translational crystallographic order, such as MCM-41, MCM-48 and SBA-15.

As used herein, the term "siliceous" takes its normal meaning known in the art. More specifically, a siliceous material is one of, relating to or containing silica or a silicate. Preferably, the encapsulating agent is silica *per se.* Optionally, however, a proportion of the silicon within the amorphous silica structure may be substituted with other elements such as boron, lead, titanium, tin, zirconium and/or aluminium. This substitution of the silica framework may be useful in adjusting the properties of the silica-based particles depending upon their specific applications. For example, addition of boron, lead, titanium, tin, zirconium and/or aluminium may result in a different refractive index.

The dye molecules are typically present within the particle in more than one area or "pocket". This beneficially maximises the dye to particle volume or weight ratio and, thus, maximises the amount of dye ultimately included in the desired end composition whilst minimizing the overall proportion of dye particles within such compositions. Irrespective of the number of areas or "pockets" of dye within the particle, each is fully surrounded or encapsulated by the encapsulating agent and, thus, held securely therein. Therefore, within the structure of the particles themselves, the encapsulating agent may be thought of as a continuous phase or matrix, whereas the dye may be thought of as comprised within a discontinuous phase. It follows that an "encapsulating agent" is an agent, which may be used to achieve this effect.

In this respect, the encapsulating agent may also be considered to be polymeric in nature because it will tend to possess crosslinking within its structure. It is preferred that the particles have as high a degree of crosslinking as possible, such that the dye is most effectively retained within the resulting particle and cannot leach therefrom. The degree of crosslinking may be observed using standard techniques such as Fourier transform infrared spectroscopy (FTIR) or solid state nuclear magnetic resonance spectroscopy (solid state NMR). Ideally and as previously mentioned, leakage of less than 5 weight %, preferably less than 2 weight %, more preferably less than 1 weight % of the total amount of dye incorporated into the particle is achieved, as determined by the methodology described herein in Example 4.

Additionally, the particles formed by spray-drying typically comprise a homogeneous distribution of the one or more dyes within the encapsulating agent. As used herein, the term "homogeneous distribution" includes particles in which the dye is homogeneously dispersed throughout the particle on a "molecular level" and the term "homogeneously distributed" should be construed accordingly. This means that the dye, typically present in one or more areas or "pocket", is not visible or discernible *via* microscopic techniques down to a range or magnification of 2nm. In other words, the particles appear as a homogeneous or single material at this level of microscopic magnification.

With reference to the dyes which may be encapsulated, reference is made to the discussion above. For the spray-drying process, the dye may be used in an unadulterated form or it may be adapted to improve its suitability to the present process. In particular, the effectiveness of some dyes containing anionic groups and a mono-valent alkali metal counter-ion, such as sodium, may be improved by ion-exchanging the metal ion with a mono-valent organic counter-iron such as ammonium or tetra-methyl ammonium.

Particularly preferred colorants or dyes include quinoline, xanthene, triarylmethane, anthracene, and monoazo based dyes.

The particles formed by spray-drying may have any shape appropriate to the end use in question. Preferably, the particles are spherical because such particles may have more predictable qualities, such as optical and rheological properties. Within a shaving application, spherical particles may also provide improved skin feel, since they may act as a lubricant by providing a ball-bearing type effect.

The particles formed by spray-drying achieve effective retention of dyes therein by an amorphous, siliceous encapsulating agent; the particles possess good chemical and physical stability, colour fastness and tint strength as well as an acceptable environmental profile. A corollary of the low dye leakage is that the surface of the silica encapsulates has similar properties to silica per se, regardless of the dye(s) incorporated therein. Thus, the particles may be reliably and effectively incorporated into compositions for use in a wide variety of applications to provide colorants, which show negligible to no leakage from the compositions into which they are incorporated and which, at the same time, provide more robust coloration to the compositions than colorants of the art. Because of this, the particles may be effectively used to provide previously unattainable dye combinations, as the individual dyes are securely held in the inventive particles.

In addition, the dye particles may be formulated into bulk colorant compositions for convenient "drop-in" use in the desired end compositions. This is particularly advantageous as end compositions currently formulated typically require specific, tailored formulation of all their individual components, including their colorants, in order to provide the correctly formulated end composition. Thus, use of colorant particles obviates the need for this repetitive, time-consuming and, therefore, uneconomic "custom" formulation by enabling the formulation of bulk end-product compositions which may then be coloured as desired using bulk colorant compositions comprising pre-determined proportions of dye particles.

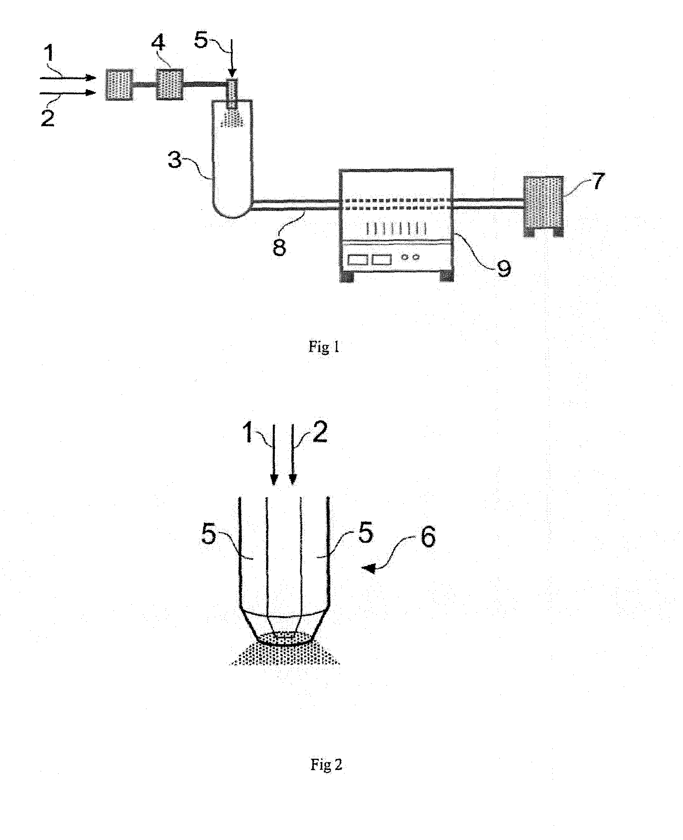

The particles may be made by an aerosol method. A suitable aerosol procedure is described with reference to Figure 1. In more detail, the encapsulating agent (1) and dye (2) are introduced in liquid form into a spray chamber (3), generally *via* means of a pump (4), together with a carrier gas (5) which is typically an inert gas such as nitrogen, or air dried by conventional methods. The liquid forms of the dye and encapsulating agent may be solutions, suspensions or dispersions, and are preferably both solutions. The liquid form of the encapsulating agent typically comprises at least one source or precursor of the siliceous encapsulating agent *per se* which, during the aerosol process, ultimately provides the desired siliceous encapsulating agent. The source of encapsulating agent may be considered to be a pre-polymer as, during aerosolisation, it will polymerise or crosslink to form the desired siliceous encapsulating agent. Preferably the siliceous precursor is organic. Suitable sources or precursors which may be used in the aerosol process to form the particles include all those conventionally used in the art to form silica, silicates and zeolites, for example. Specific examples of useful silica precursors include tetramethylorthosilicate (TMOS), tetraethylorthosilicate (TEOS), tetrapropylorthosilicate (TPOS), tetraisopropylorthosilicate (TiPOS), tetrabutylorthosilicate (TBOS), silicic acid which may for example be modified with cations such as sodium or ammonium so that it is provided in the form of sodium silicate (also known as waterglass) or ammonium silicate. TEOS is a particularly preferred source of silica from a safety perspective, because the by-product of the process is ethanol (not methanol, as is the case for TMOS).

As is easily determinable and generally known by a skilled person, approximately one third of the weight of precursor is transformed into particles. For example, silica (SiO₂) has a molecular weight of 60g/mole and TEOS has a molecular weight of 208g/mole, so the weight of silica produced is 60/208 or 0.29 times the amount of TEOS. For TMOS the value is 0.39 (the molecular weight of TMOS is 152g/mole).

In addition, the amount of dye encapsulated within the particles is easily calculable by a skilled person. For example, if one wants a 10% dye loading, then, bearing in mind how much precursor one needs, as discussed above, it is a straightforward matter to calculate the amount of dye needed (a precursor solution of 10.4g of TEOS and 0.33g of dye, for example, yields silica particles comprising 90% silica (3g silica) and 10% dye (0.33g dye)).

The solvent used will depend upon the hydrophobicity of the starting material. TEOS, TMOS and TPOS are hydrophobic and are therefore generally solubilised in an essentially non-aqueous material, for example as an alcoholic solution such as a solution in ethanol, methanol, n-propanol, iso-propanol and/or a butanol, i.e. n-butanol, 2-butanol, iso-butanol or tert-butanol. Alternatively, a solution in acetone or one or more other conventional solvents, for instance, may also be employed. Silicic acid and the silicates are hydrophilic so may be dissolved in hydrophilic solvents such as water. The amounts of solvent used are readily determinable by a skilled person - the lower limit is, in practice, determined by the solubility parameters of the starting material and the upper limit is a practical one - the more solvent one uses, the smaller the final particles and the smaller the production capacity.

Although the solvent may be non-aqueous, some water is, nevertheless, necessary in order to hydrolyse the precursor, such as TEOS, to silicic acid prior to aerosolisation. Hydrolysis prior to aerosolisation is important to minimise the number of pores in the resulting particles, thereby minimising leakage of encapsulated dye. Typically, it is preferred that the aqueous portion be an acidic solution. The pH will be more than 1 and less than 7 and is advantageously approximately 2, as this is at or near the iso electric point of silica itself. The pH of the precursor liquid form may be adjusted as desired using techniques conventionally used in the art, for example by addition of acid. A preferred acid used for this purpose is hydrochloric acid. Water of the requisite pH may be introduced as a solvent for the silica precursor or as a solvent for the dye, as discussed below.

The dyes incorporated into the particles are provided to the aerosol process in acidic, basic or neutral liquid form. Preferably the one or more dyes is provided in the form of a solution in one or more solvents conventionally used in this field, preferably water or an alcohol such as methanol, ethanol, n-propanol, iso-propanol or a butanol (as previously defined), and particularly preferably ethanol or water. Highly preferably, the dye is provided in the form of an aqueous solution, and conveniently as an aqueous ethanolic solution. It may be necessary to aid dissolution of the active in the chosen solvent by providing it in the form of a salt, for instance those formed with commonly-used cations such as sodium, ammonium or potassium, or by adjusting the pH of the mixture of dye and solvent, again by conventional methods as previously described.

As mentioned above, it is desirable that, in creating the aerosol to form the particles, at least one of the liquid forms of encapsulating agent and dye should be aqueous. It is usually preferred that the dye be provided in aqueous liquid form, whereas the siliceous precursor is typically provided in non-aqueous form. The presence of water in the reaction medium aids pre-hydrolysis of the silica precursor which, in turn, aids subsequent polymerisation of the precursor to form the desired encapsulating agent comprising a low number of pores. The liquid forms of the dye and precursor are preferably mixed together prior to entry into the aerosol chamber for this purpose.

In addition and as mentioned above, if the siliceous encapsulating agent incorporates other inorganic materials in its structure in addition to silica, these may also be provided to the aerosol process in liquid forms of conventional sources of such material. If it is desired to include titanium dioxide, for example, it may be appropriate to include a solution of tetraethoxytitanate dissolved in an appropriate solvent, such as ethanol.

A suitable aerosol procedure is described with reference to Figure 1 in which the encapsulating agent (1) and dye (2) are mixed, then introduced in liquid form into a spray chamber (3), generally *via* means of a pump (4), together with a carrier gas (5) which is typically an inert gas such as nitrogen, or air dried by conventional methods.

Typically a spray nozzle (6) such as that shown in Figure 2 is used in the aerosol process, whereby the dye (2) and agent (1) are introduced through a central tube and the carrier gas (5) is introduced through an outer tube of the nozzle. This type of nozzle is conventionally known as a "two-flow spray nozzle", however, other nozzle types commonly used in creating aerosols may also be employed. A two flow nozzle is preferred as the carrier gas flow cuts across or dissects the central flow of dye and encapsulating agent, thus facilitating more effective formation of spray droplets comprising the encapsulating agent and dye. Whilst the apparatus shown in Figures 1 and 2 illustrates a downwardly-spraying nozzle, it will be appreciated that all conventional types of aerosol apparatus including upwardly spraying apparatus may be conveniently used in the aerosol process. Indeed, so-called "spray up" systems may be preferred where it is desirable to fractionate particles of different sizes directly from the spray chamber, for instance.

The droplets formed in the spray chamber (3) are typically held in the chamber for a residence time in the range of greater than 0 up to about three minutes. Residence time may affect the porosity and, to a limited extent, the size of the resulting particles. For instance, for an average particle size of approximately 3-5 µm and a minimum porosity, a residence time of approximately 10 seconds maybe conveniently employed. When present in the spray chamber, the encapsulating agent undergoes crosslinking within itself, thus forming droplets of a secure cage-like structure or network within which the dye is securely held. In addition, of course, the solvents evaporate. Typically the particles have a diameter which is half that of the droplets sprayed into the spray chamber (3).

The droplets are then removed from the spray chamber in a conventional manner for instance *via* means of a pressure differential created by a pump (7) located at the end of a tube (8), into which the droplets pass from the spray chamber. Generally, the tube (8) into which the droplets pass is heated to a temperature which will effect drying of the particles for instance *via* means of a heater (9). Typically, a temperature in the range of approximately 150-250 °C is employed. Heating of the droplets in this way promotes condensation and, thus, further crosslinking of the siliceous encapsulating agent, preferably ultimately resulting in the formation of substantially fully crosslinked polymer-encapsulated dye particles.

The particles made in the aerosol process are typically dried by any means conventionally known in the art, such as a heater, either before or after their recovery from the aerosol apparatus which is, again, achieved in a conventional manner.

Optionally, the particles may undergo a subsequent washing process; in order to ensure that all of the dye is securely encapsulated within the particles and that none remains at the surface of the particles. Conventional washing agents or solvents such as water, alcohols or acetone may be used for this purpose, the choice of washing agent typically being dependent upon the solubility characteristics of the relevant dye(s).

The conditions under which particles of the invention are produced by the aerosol process are not critical. Accordingly, aerosolisation may be performed under temperature, pressure and other conditions as desired by the skilled person in this technical field. Typically and conveniently, however, aerosolisation is performed under ambient temperature and pressure conditions, i.e. at room temperature of approximately 18-25°C, and at a pressure of approximately atmospheric pressure. However, it will be appreciated that lower or higher temperatures and pressures may be employed as desired. In addition, it is not essential to exclude humidity from the aerosol apparatus. As such, the relative humidity (RH) within the aerosol apparatus does not need to be monitored but, under ambient conditions, is typically less than 50 %, as measured by conventional techniques.
The particles have a specific surface area of 0.1m²/g to 25m²/g, preferably 0.5m²/g to 5m²/g, more preferably 0.5m²/g to 3.5m²/g. In addition, the particles have a specific internal pore volume of 0.001 to 0.03 cm³/g, preferably 0.001m³/g to 0.011cm³/g. Surface areas and pore volumes are determined using nitrogen porosimetry using nitrogen at a temperature of -196°C or 77K. The samples are evacuated at 120-150°C for at least 4-6 hours to remove adsorbed water from the pores, and sample sizes are preferred to be around 0.5g. Otherwise standard procedures for collecting high quality N₂ isotherm data should be followed. The pore volumes are cumulative pore volumes for internal pores less than 50 nm in diameter and are determined using the "Barret-Joiner-Halenda" method.

The dye-loaded particles may be provided with a hydrophilic coating to improve the particles' dispersion in an aqueous carrier medium. Any hydrophilic coating known to the skilled person may be used.

The dye-loaded particles may be provided with a hydrophobic coating to improve the particles,' dispersion in hydrophobic carrier medium. Advantageously, the hydrophobic coating may be made by applying a mixture of one or more of the following materials and isopropyl alcohol onto the dye-loaded powder and drying at 150°C for 3 hours: reactive organo-polysiloxane, polyolefin (including polyethylene and polypropylene), hydrogenated lecithin and salts thereof, N-acylamino acid and salts thereof and dextrin fatty acid esters. Preferably, the reactive organo-polysiloxane comprises organo hydrogen polysiloxane, triorgano siloxy silicic acid and organopolysiloxane modified at both terminal ends with trialkoxy groups. Commercially available materials falling into the category of reactive organo-polysiloxanes include KF-99, KF-9901, KF-7312F, KF-7312-J, KF-7312K, KF-9001, KF-9002, X-21-5249 and X-21-5250 manufactured by the Shin-Etsu Chemical Company Ltd; PH-1107, DC593, BY-11-015, BY-11-018 and BY-11-022 manufactured by Dow Coming Toray Silicone Co. Ltd.; TSF484, TSF483 and TSF4600 manufactured by Toshiba Silicone Co. Ltd.; FZ3704 and AZ6200 manufactured by Nippon Unicar Co. Ltd.

The hydrophobic coating is not limited to those described in the preceding paragraph and alternative hydrophobic coatings known to the skilled person may be employed instead. Such coatings may include trialkoyl isopropyl titanate, preferably triisostearoyl isopropyl titanate and perfluoro coatings, preferably polyperfluoroethoxymethoxy PEG-2 phosphate.
In addition to the hydrophobic coating, the dye-loaded particles may be provided with a coating of organo-functionalised silicone fibrils such as those described in EP 1 602 352. Such fibril coatings may reduce or prevent agglomeration of dye-loaded particles.

Some coatings may both provide hydrophobic properties and exhibit fibrils to avoid flocculation. Commercially available coatings falling into this category include KF9908 (Triethoxysilylethyl Polydimethylsiloxyethyl Dimethicone), KF9909 (Triethoxysilylethyl Polydimethylsiloxyethyl Hexyl Dimethicone) and KP575 (Acrylate/Tridecyl Acrylate/Triethoxysilylproplyl Methacrylate/Dimethicone Methacrylate Copolymer) from the Shin Etsu Co Ltd.

The non-foaming shaving compositions of the present invention may be in any suitable delivery form, such as emulsions, including oil-in-water, silicone-in-water, water-in-oil and water-in-silicone emulsions and multiple emulsions; oils; gels; aerosols and mousses.

Non-foaming shaving compositions according to the invention may comprise oil. The oil may be selected from the group consisting of volatile oils, non-volatile oils and mixtures thereof

As used herein, the term "non-volatile" when employed in relation to an oil includes oils that fulfill at least one of the following definition: (a) the oil exhibits a vapour pressure of no more than about 0.2 mm Hg at 25°C and one atmosphere pressure; (b) the oil has a boiling point at one atmosphere of at least about 300°C. As used herein, the term "volatile" when employed in relation to oils includes materials that are not "non-volatile" as previously defined herein.

Any non-volatile oil adhering to the above definition may be included in non-foaming shaving compositions according to the invention. Such non-volatile oils may include silicone oils, both functionalised and non-functionalised, hydrocarbon oils and mixtures thereof.

Volatile oils which may be included in shaving compositions according to the invention may include silicone oils, both functionalised and non-functionalised, hydrocarbon oils and mixtures thereof. The oils may be saturated or unsaturated, have a straight or branched chain or a cyclic structure. Examples of volatile hydrocarbons which may be incorporated into shaving compositions according to the invention include polydecanes such as isododecane and isodecane (e.g., Pamethyl-99A which is available from Presperse Inc.) and the C₇-C₁₅ isoparaffins (such as the Isopar Series available from Exxon Chemicals). Examples of volatile silicone oils which may be incorporated into shaving compositions according to the invention include cyclic volatile silicones corresponding to the formula: 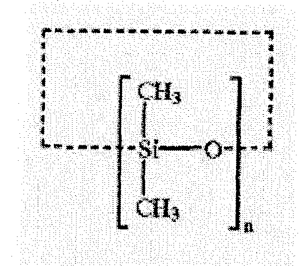 wherein n is from about 3 to about 7 and linear volatile silicones corresponding to the formula:

(CH₃)₃Si-O-[Si(CH₃)₂-O]ₘ-Si(CH₃)₃

wherein m is from about 1 to about 20 preferably from 3 to 12.

Preferably, the cyclic volatile silicone is cyclopentasiloxane or cyclohexasiloxane. Linear volatile silicones generally have a viscosity of less than about 5 centistokes at 25°C; cyclic silicones generally have viscosities of less than about 10 centistokes at 25°C.

Examples of commercially available volatile silicone oils include the following cyclomethicones: Dow Coming 200, Dow Coming 244, Dow Coming 245, Dow Coming 344, and Dow Corning 345 (commercially available from Dow Corning Corp.); SF-1204 and SF-1202 Silicone Fluids (commercially available from G. E. Silicones), GE 7207 and 7158 (commercially available from General Electric Co.); and SWS-03314 (commercially available from SWS Silicones Corp.). Other examples of commercially available methyl silsesquioxanes available as TMF 1.5 fluid from Shin-Etsu Chemical Co; SILCARE SILICONES, for example phenyl substituted silsesquioxanes available as Silcare 15M60, n-Octyl substituted silsesquioxanes available as Silcare 31M60 and 31M50, hexyl methicone, caprylyl methicone and lauryl methicone available as Silcare 41M10, 41M15 and 41M20 respectively from Clariant.

Preferred examples of linear dimethicones user include DC200 5cst, DC1630 and DC 5-2117, More preferably, the linear dimethicone comprises DC 5-2117.

The shaving compositions of the present invention can also comprise a thickening agent, which may be a water phase thickening agent or an oil phase thickening agent.

Non-limiting classes of water phase thickening agents comprise carboxylic acid polymers, crosslinked acrylate copolymers, polyacrylamide polymers or mixtures thereof
(i) Carboxylic Acid Polymers These polymers are crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. Examples of carboxylic acid polymer thickeners useful herein are those selected from the group consisting of carbomers (available as the Carbopol 900™ series from B.F. Goodrich e.g. Carbopol 954™), acrylates/C10-C30 alkyl acrylate crosspolymers (commercially available as Carbopol 1342™, Carbopol 1382™, Pemulen TR-1™, and Pemulen TR-2™, from B.F. Goodrich) and mixtures thereof.
(ii) Crosslinked Acrylate Copolymers These polymers comprise a blend of a water soluble anionic acrylic monomer, a water soluble non-ionic acrylate monomer and a bifunctional monomeric cross-linking agent. Suitable water soluble anionic acrylic based monomers include acrylic acrid, methacrylic acid and mixtures thereof Suitable water-soluble non-ionic acrylate-based monomers include acrylamide, methacrylamide, N-vinyl pyrolidone, water-soluble hydroxy-substituted acrylic or methacrylic esters or mixtures thereof. Suitable bifunctional monomeric cross-linking agents include di-, tri- and tetraethylenically unsaturated materials such as methylene bis acrylamide, divinylpyrroline and allyl (meth) acrylate or mixtures thereof Commercial examples of co-polymer compositions suitable for use herein include the co-polymer compositions commercially available from BASF Corp. under the tradename Luvigel™ EM and the co-polymer compositions available from CIBA Speciality Chemicals, Macclesfield, UK, under the tradename Salcare SC91™.
(iii) Polyacrylamide Polymers Also useful herein are polyacrylamide polymers, especially anionic polyacrylamide polymers including substituted branched or unbranched polymers. These polymers can be formed from a variety of monomers including acrylamide and methacrylamide which are unsubstituted or substituted with one or two alkyl groups (preferably C₁ to C₅). Preferred are acrylate amide and methacrylate amide monomers in which the amide nitrogen is unsubstituted, or substituted with one or two C₁ to C₅ alkyl groups (preferably methyl, ethyl, or propyl), for example, acrylamide, methacrylamide, N-methacrylamide, N-methylmethacrylamide, N,N-dimethylmethacrylamide; N-isopropylacrylamide, N-isopropylmethacrylamide, and N,N-dimethylacrylamide. These polymers have a molecular weight greater than about 1,000,000 preferably greater than about 1,5000,000 and range up to about 30,000,000. Most preferred among these polyacrylamide polymers is the anionic polymer given the CTFA designation polyacrylamide and isoparaffin and laureth-7, available under the tradename Sepigel 305 from Seppic Corporation (Fairfield, NJ).

Suitable oil phase thickening agents can be selected from the group consisting of silicones, waxes, clays and mixtures thereof Non-limiting examples of these thickening agents are described below.

Suitable silicones include alkyl siloxane gellants, high molecular weight dimethicones (fluids greater than 1000 mPas), and high molecular weight alkyl, hydroxyl, carboxyl, amino, and/or fluoro- substituted dimethicones (fluids greater than 1000 mPas). Referred silicone gellants are described in US patent 5,654,362 and 5,880,210, and include cyclomethicone and dimethicone crosspolymers (e.g., Dow Corning 9040).

A variety of additional optional ingredients may be incorporated into the compositions of the present invention. Non-limiting examples of these additional ingredients include additional skin care actives such as precursors of the glycosaminoglycans, including, but not limited to acetylglucosamine and glucuronic acid; peptides (such as, palmitoyl-lys-thr-thr-lys-ser]), farnesol, bisabolol, phytantriol, urea, guanidine (e.g., amino guanidine); vitamins and derivatives thereof such ascorbic acid, vitamin A (e.g., retinoid derivatives such as retinyl palmitate or retinyl proprionate), vitamin E (e.g., tocopherol acetate), vitamin B₃ (e.g., niacinamide) and vitamin B₅ (e.g., panthenol) and the like and mixtures thereof; anti-acne medicaments (resorcinol, salicylic acid, and the like; emollients (e.g., fatty esters such as isopropyl myristate, decyl oleate, 2-ethylhexyl palmitate, PEG-7 glyceryl cocoate, and glyceryl linoleate, propoxylated fatty ethers such as PPG-10 cetyl ether and PPG-11 stearyl ether, di-and triglycerides such as lecithin and caprylic/capric triglyceride, vegetable oils, and similar materials); antioxidants (e.g., phytosterols, lipoic acid); flavonoids (e.g., isoflavones, phytoestrogens); skin soothing and healing agents such as aloe vera extract, allantoin and the like; skin whitening agents, such as but not limited to, ascorbic acid and its derivatives, including sodium and magnesium ascorbyl phosphate; self-tanning agents, such as dihydroxyacetone; chelators and sequestrants; fatty alcohols (e.g. myristyl, lauryl and stearyl alcohol and octyl dodecanol), propellants and agents suitable for aesthetic purposes such as essential oils, fragrances, skin sensates, opacifiers, aromatic compounds (e.g., clove oil, menthol, camphor, eucalyptus oil, and eugenol).

Although the shaving compositions according to the present invention are non-foaming, it may be necessary for them to comprise a surfactant. However, as the presence of surfactants in a composition intended for topical use may result in a drying effect on the skin, or irritation, it may be preferable to limit the presence of surfactant to a level of from greater than 0% to 8%, more preferably from greater than 0% to 5%, even more preferably 0%.

Humectants which may be included in shaving compositions according to the invention include polyhydric alcohols such as glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerine and mixtures thereof. Most preferably the humectant comprises glycerine.

Although it is possible to include additional colorants in the compositions according to the invention, it is preferred that the present compositions comprise less than 0.5%, preferably less than 0.1% by weight of the composition and more preferably no free organic dye.

Non-foaming shaving compositions according to the invention may be comprised within a shaving device and be dispensed directly from the shaving device onto a user's skin before, during or after use of the razor. According to this aspect of the invention, a shaving device is provided, the shaving device comprising a reservoir containing the non-foaming shaving composition and means for dispensing the formulation onto the skin of a user. Such dispensing means are known in the art and need not be defined further here.

### Examples

The present invention will now be described in more detail with reference to the following non-limiting example(s):

### Example 1: Preparation of Silica loaded with Tartrazine (FD&C Yellow No.5)

As a first step to synthesising sodium tartrazine-containing silica, the dye (commercially available from Sigma as T0388-100G (CAS# 1934-21-0) was ion-exchanged using a column with ion-exchanging resin (type Dowex 50Wx8 commercially available Dow Chemical Comp., Michigan, USA. This was necessary because the use of commercial Tartrazine induces flocculation of the tetraethylorthosilicate/ethanol/hydrochloric acid (TEOS/EtOH/HCI) encapsulating agent mixture.

### Column Preparation

The column was loaded with 317 g Dowex 50Wx8 to obtain a 400ml bed volume.

Step 1 - Washing: to remove residual sodium cations, the column was eluted with 2.5 1 deionized water over 5 to 10 minutes at pH 6.

Step 2 - Reconditioning: to remove bound sodium cations the column was washed through with four batches of 400 ml 7% HCl. The contact time of HCl on the column was 45 minutes. Step 3 - Washing: as for Step 1.

Step 4 - Charging: as for Step 2, but it was performed using 7% ammonium chloride (NH₄Cl) instead of HCl.

Step 5 - Washing: as for Step 1, the purpose being to remove excess ammonium cations.

### Ion Exchange of Dye

A 10% dye solution of sodium tartrazine was prepared in an acidic solution of water and ethanol and was eluted through the column. This solution was used to produce Tartrazine-containing silica in the subsequent procedure.

### Spraying of Silica with Yellow Dye

Two batches of coloured silica were each prepared using 10.4 g TEOS, 5.4 g of HCl with a pH of around 1.25 and 12.0 g of ethanol. The components were mixed together and the mixture was left stirring for 30 minutes. Theoretically, such a mixture should give 3 g of silica after aerosolisation. The calculation of the amount of Tartrazine solution to add was based on this theoretical amount of silica. The ion-exchanged Tartrazine solution was then mixed with 4 g of ethanol.

The two resulting mixtures were then blended together, the pH was adjusted to pH 2.0 using 1M HCl and the blend was left under stirring for a further 10 minutes. The blend was then aerosolized and spray-dried as follows:

The starting solution blend is pumped at a constant rate of 3ml/minute using a peristaltic pump to the centre flow outlet of a coaxial two-flow spray nozzle of a spray tower. At the same time, compressed air is pumped at 20litres/minute (at STP) to outer annular outlet located coaxially around the centre flow outlet. The centre flow outlet diameter is 1mm; the outer diameter is 1.5mm. The spraying was such that a turbulent mixture was propelled into the spray chamber, which was retained at ambient temperature. Afterwards, the mixture was heated to 220°C to induce cross-linking and drying of the particles.

### Washing of Particles

The particles were washed with de-ionized water, at a rate of 200 ml water per 1 g of particles as follows: 5 g of particles were placed into a plastic bottle and 1000 ml of water were added. The mixture was left under stirring for 5 minutes and it was then centrifuged for 10 minutes at 3500 rpm. The sediment was then separated from the supernatant fluid.

### Separation of Small Particles

The sediment from the centrifuged mixture was mixed with 1000 ml water in a beaker and left to settle for two days. The resulting supernatant fluid was then pumped into another beaker using a roll pump. Once the supernatant had been pumped into the other beaker, it was centrifuged at 3500 rpm for 20 minutes and the resulting sediment was separated using a standard separation technique from the liquid. The resulting particles were then dried in an oven at 50°C.

### Particle Size Measurement

The size of the resulting particles was measured using a Malvern Master Sizer 2000 apparatus, which measures particle size *via* light scattering. Particle size distribution is as follows:
d(0.1): 0.308 µm (10% of the particles have a size lower than the volume averaged value given)
d(0.5): 0.539 µm (50% of the particles have a size lower than the volume averaged value given)
d(0.9): 0.953 µm (90% of the particles have a size lower than the volume averaged value given)

### Example 2: Reparation of Silica loaded with Amaranth (Acid Red No.27)

This red dye is soluble in water but it is not soluble in ethanol. It was not necessary to ionexchange the aqueous solution (as was done for Tartrazine in Example 1), however, as it did not flocculate when it was blended with the TEOS mixture. Also, even though the dye was insoluble in ethanol, it was still possible to use it directly by increasing the water proportion in the aerosol mixture.

### Sample Reparation

Two batches of coloured silica were prepared using 10.4 g TEOS, 5.4g of HCl of pH 2 and 12.0 g of ethanol. The components were mixed together and the mixture was left stirring for 30 minutes. Theoretically, the mixture would give 3 g of silica after the aerosolisation. Thus, the calculation of the amount of Amaranth solution (obtained from Sigma has catalogue number A1016-100G (CAS#915-67-3)) to add was based on this amount of silica. This was as follows: 0.3 g of Amaranth powder plus 10.0 g of HCl (pH 2). The two mixtures were mixed together and left to stir for 10 minutes. The mixture was subsequently spray dried, heated, washed, the particles separated and size measured as in example 1. Particle size distribution is as follows:
d(0.1): 0.322 µm
d(0.5): 0.592 µm
d(0.9): 1.130 µm

### Example 3: Preparation of Silica loaded with Erioglaucine (FD & C Blue No. 1)

This blue dye is soluble in water and ethanol and it was not necessary to ion exchange the solution.

### Sample Preparation

Two batches of coloured silica were prepared each using 10.4 g TEOS, 5.4 g HCl (pH2) and 8.0 g of ethanol. The components were mixed together and the mixture was left under stirring for 30 minutes. Theoretically, the mixture would give 3g of silica after the aerosolisation. Thus, the calculation of the amount of Erioglaucine solution to add was based on this amount of silica. This was as follows: 0.3 g of Erioglaucine powder (obtained from Sigma/Aldrich, catalogue# 861146-25G (CAS#3844-45-9)) plus 2.0 g of HCl (pH 2) in 3 g ethanol. The two mixtures were mixed together and left to stir for 10minutes. The mixture was subsequently spray dried, heated, washed, the particles separated and size measured as in example 1. Particle size distribution is as follows:
d(0.1): 0.327 µm
d(0.5): 0.59 µm
d(0.9): 1.130 µm

### Example 4: Dye Release/Leakage Experiments

### Examples 4A

For the products of each of Examples 1, 2 and 3, 0.2 g of the particles loaded with dye were placed into a centrifuge tube and 10 ml of a water/propanol (1:1) mixture was added. The tube was shaken for two minutes and centrifuged. The supernatant fluid was separated from the sediment and collected in a bottle. This operation was repeated five times. The supernatant fluid from all five extractions was mixed and analysed using a UV spectrometer The results are provided in the following table (3).

| **Table 3** | | | |
|---|---|---|---|
| Wash number | Release Yellow, wt% | Release Red, wt% | Release Blue, wt% |
| 1 | 0.25 | 0.36 | 0.1299 |
| 2 | 0 | 0.027 | 0.0196 |
| 3 | 0 | 0 | 0.0091 |
| 4 | 0 | 0 | 0 |
| 5 | 0 | 0 | 0 |
| Sum over five washes | 0.25 | 0.387 | 0.1586 |

The results show that the release of the dyes into the water/propanol mixture was extremely low.

### Example 4B

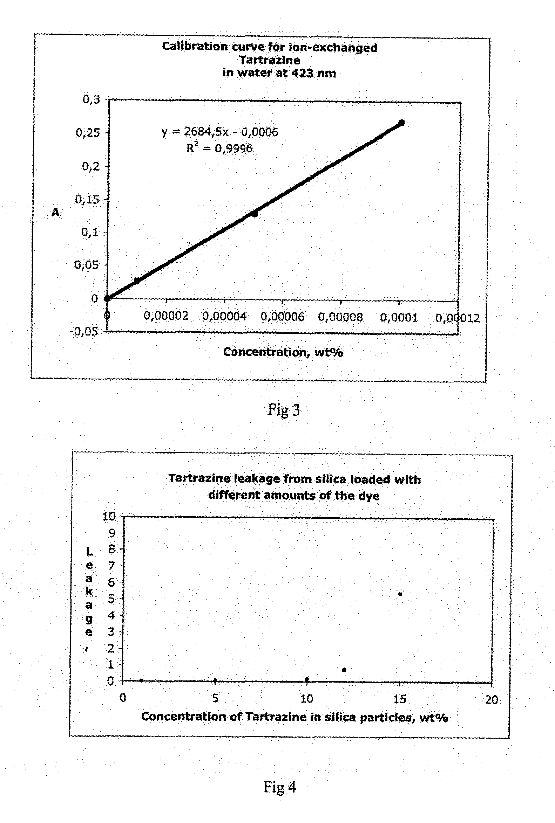

The leakage of Tartrazine from silica loaded with different amounts of dye was investigated as follows. In turn, 1 g of particles loaded with 1%, 5%, 10%, 12% and 15% respectively of Tartrazine was placed into a bottle and 100 g of water was added. The mixture was left under stirring for 3 hours, after which time, a 5 ml portion was extracted from every bottle using a syringe. This portion was filtered with a membrane filter (0.45 µm) and analyzed in an UV-VIS spectrophotometer. The leakage in wt% was calculated for every sample with help of the calibration curve presented as Figure 3, which shows the calibration for ion-exchanged Tartrazine in water at 423 nm.

The results are presented in the following table (4).

| **Table 4** | |
|---|---|
| **Tartrazine Concentration (wt%)** | **Leakage (wt%)** |
| 1 | 0.044 |
| 5 | 0.0528 |
| 10 | 0.155 |
| 12 | 0.727 |
| 15 | 5.37 |

These results are presented in Figure 4, and show that dye leakage, in the case of Tartrazine-loaded particles, substantially increases at greater than 12 wt% Tartrazine concentration within the particles.

### Examples 5 and 6: Aqueous shaving compositions

| **Table 5** | | |
|---|---|---|
| **Example #** | **5** | **6** |
| **Ingredient** | **% w/w** | **% w/w** |
| | Shave gel | Gel |
| Water | Qs | Qs |
| Carbopol Ultrez 20 | 1.5 | 0.0 |
| Carbopol Ultrez 21 | 0.0 | 0.4 |
| Sodium hydroxide solution (50% w/w) | 0.8 | 0.0 |
| Cocoamidopropylbetaine (Tegobetaine F50) | 3.0 | 0.0 |
| Glycerin | 3.0 | 6.0 |
| Dipropylene glycol | 0.0 | 5.0 |
| Cyclopentasiloxane (DC245) | 0.0 | 4 |
| DC1503 silicone gum | 0.0 | 2 |
| Tospearl powder (CF 600) | 0.0 | 2 |
| Disodium EDTA | 0.1 | 0.025 |
| Glydant Plus | 0.0 | 0.3 |
| Benzophenone-4 | 0.15 | 0.0 |
| Encapsulated FD&C Yellow 5 particles | 1.0 | 0.0 |
| Encapsulated FD&C Blue 1 particles | 1.0 | 1.0 |

| | | |
|---|---|---|
| 1. Carbopol: Supplied by Noveon 2. Tegobetaine: Supplied by Evonik Degussa 3. DC245 and DC 1503 : Supplied by Dow Coming 4. Tospearl: Supplied by GE Silicones | | |

A water phase is prepared by admixing all the water soluble ingredients according to Table 5, except sodium hydroxide and surfactant, in water. The Carbopol is sprinkled on the surface of the batch with slow agitation and allowed to wet out. For Example 5, a second premix is prepared by admixing of the surfactant and sodium hydroxide solution plus some additional water if required. For Example 6, a second pre-mix is prepared containing the silicones and silicone powder. The second pre-mix is added to the first and agitation is increased to produce a smooth, uniform appearance. Finally the encapsulated dye is added and incorporated into the final product. The product can then be prepared for packaging.

### Example 7: Non-aqueous shaving composition

| **Table 6** | |
|---|---|
| **Example #** | **7** |
| **Ingredient** | **% w/w** |
| | Oil |
| Dimethicone | Qs |
| Cyclopentasiloxane | 20.0 |
| Dimethiconol | 10.0 |
| Encapsulated FD&C Blue 1 particles (hydrophobically modified) | 2.0 |

The product is prepared by co-mixing the ingredients according to Table 6 with a moderate level of shear. The final product can then be packaged.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "4omm" is intended to mean "about 40mm".

## Claims

1. A non-foaming shaving composition comprising:
(i) Particles, each particle comprising at least one dye encapsulated by at least one siliceous encapsulating agent; and
(ii) A dermatologically acceptable carrier

2. A non-foaming shaving composition according to claim 1, wherein the dye is homogenously distributed throughout the particles.

3. A non-foaming shaving composition according to any preceding claim, wherein the particles are present in the composition in a concentration range of from 0.05% to 10%, preferably from 0.1 % to 5%, more preferably 0.1% to 2%.

4. A non-foaming shaving composition according to any preceding claim, wherein the particle size is in the range of greater than 0 to 10µm, preferably in the range of greater than 0 to 5µm, more preferably from greater than 0 to less than 1 µm and even more preferably from 10nm to less than 1 µm.

5. A non-foaming shaving compositions according to any preceding claim, wherein the particles further comprise a hydrophobic coating, preferably selected from: reactive organo-polysiloxanes, polyolefins, hydrogenated lecithin and salts thereof, N-acylamino acid and salts thereof and dextrin fatty acid esters, more preferably selected from: organo hydrogen polysiloxane, triorgano siloxy silicic acid and organopolysiloxane modified at both terminal ends with trialkoxy groups.

6. A non-foaming shaving composition according to any preceding claim, wherein the dye is present in the particles in a concentration range of from 3% to 20%, preferably 5% to 15%, more preferably 8% to 12% by weight of the particle.

7. A non-foaming shaving composition according to any preceding claim, wherein the one or more dyes are selected from: caramel, cochineal, beta carotene, guanine, henna, quinoline, xanthene, triarylmethane, anthracene, monoazo dyes or derivatives thereof, preferably quinoline, xanthene, triarylmethane, anthracene or monoazo dyes, more preferably quinoline dyes.

8. A non-foaming shaving composition according to any preceding claim, wherein the siliceous encapsulating agent comprises one or more of titanium, tin, zirconium, aluminium, boron and lead.

9. A non-foaming shaving composition according to any preceding claim, wherein the encapsulating agent of the particles is silica.

10. A non-foaming shaving composition according to any preceding claim, wherein the composition comprises a surfactant in a concentration range of from greater than 0% to 8%, preferably greater than 0% to 5%

11. A non-foaming shaving composition according to any of claims 1 to 9, wherein the composition comprises no surfactant.

12. A shaving device comprising a reservoir containing the formulation according to any preceding claim and means for dispensing the formulation onto the skin of a user.

13. Shaving method comprising the following steps:
(a) topically applying a non-foaming shaving composition according to any of claims 1 to 11 I to an area of skin to be shaved;
(b) identifying areas of skin that have not been shaved by means of the colour bestowed by the particles;
(c) shaving identified areas of skin which have not been shaved.
